# FASCICULE DE BREVET EUROPEEN

(11) **EP 2 475 332 B1**
(45) Date de publication et mention de la délivrance du brevet: **30.04.2014**
(21) Numéro de dépôt: 10762718.4
(22) Date de dépôt: 09.09.2010
(51) Int. Cl.: A61F 2/28, A61L 27/04, A61L 27/56

(54) **STRUCTURE POREUSE À MOTIF CONTRÔLÉ, RÉPÉTÉ DANS L'ESPACE, POUR LA RÉALISATION D'IMPLANTS CHIRURGICAUX**
PORÖSE STRUKTUR MIT EINEM GESTEUERTEN, IM RAUM WIEDERHOLTEN MUSTER ZUR HERSTELLUNG CHIRURGISCHER IMPLANTATE
POROUS STRUCTURE HAVING A CONTROLLED PATTERN, REPEATED IN SPACE, FOR PRODUCING SURGICAL IMPLANTS

(30) Priorité: 09.09.2009 FR 0904314
(43) Date de publication de la demande: 18.07.2012
(73) Titulaire: OBL (Société Anonyme), 92320 Chatillon (FR)
(72) Inventeur: LONGEPIED, Patrice, F-92330 Sceaux (FR); DUBOIS, Guillaume, F-92170 Vanves (FR)
(74) Mandataire: Blaseby, Matthew Peter
(86) Numéro de dépôt international: PCT/FR2010/000614
(87) Numéro de publication internationale: WO 2011/030017

(56) Documents cités:
- EP-A2- 1 683 593
- WO-A1-2009/048314
- DE-U1-202006 015 415
- US-A1- 2005 112 397
- US-B1- 6 709 739

## Description

La présente invention est relative à une structure poreuse à motif contrôlé, répété dans l'espace, dans les trois dimensions, ladite structure poreuse permettant la réalisation d'implants chirurgicaux destinés à combler les défects osseux.

Il est connu d'utiliser des implants pour pallier les absences totales ou partielles d'os ou les défauts d'os chez l'homme ou chez l'animal, ces implants étant réalisés de sorte à présenter une structure poreuse imitant le plus possible celle de l'os.

Les pores de l'implant permettent en effet aux cellules osseuses de se multiplier dans ledit implant, donc d'assurer un meilleur transfert de charge entre l'os et l'implant, ce qui a pour effet d'ancrer ce dernier solidement dans les parties voisines saines de l'os à réparer ou à combler.

Il s'ensuit par conséquent une guérison plus rapide.

Les implants classiques sont faits en titane, matériau dont on sait qu'il est biocompatible.

On connait déjà de nombreux procédés de réalisation de tels implants en titane dont la structure est poreuse.

Le premier procédé a consisté à réaliser un implant dont la forme elle-même et la géométrie correspondent à la partie de l'os à combler ou à réparer.

Selon ce premier procédé, on réalise un prototype en cire dont la géométrie correspond exactement à la partie de l'os à combler ou à réparer.

Ce prototype est ensuite immergé dans un matériau réfractaire et cet ensemble est chauffé afin de détruire la cire.

Le matériau réfractaire persistant est ensuite fritté à haute température afin d'obtenir un moule. Il suffit alors de couler le titane en fusion dans ce moule, lequel sera ensuite détruit par des dissolvants chimiques qui n'attaquent pas le titane.

Un autre procédé voisin consiste à introduire des microbilles en titane dans le moule réalisé ainsi qu'il vient d'être dit et à solidariser ces microbilles entre elles par électro-étincelage.

Le défaut de ces procédés anciens est toutefois que, si les implants ainsi réalisés ont chacun la forme désirée, ils ne sont toutefois pas ou peu poreux et, en toute hypothèse, il est impossible de dicter la taille des pores et, par voie de conséquence, le taux de porosité de l'implant.

Un procédé amélioré est basé sur l'utilisation d'une pâte constituée de poudre de titane, d'une solution aqueuse de méthyl-cellulose et d'acide stéarique. Cette pâte est déposée en couches superposées au fond d'un bac afin de former la géométrie de la pièce voulue. L'ensemble est ensuite séché, puis fritté sous vide à haute température, afin d'obtenir la pièce finie (Biomatérials 27 (2006) 1223-1235).

Un procédé voisin est basé sur l'utilisation d'une pâte constituée de poudre de titane mélangée à du bicarbonate d'ammonium. Le mélange est comprimé dans un moule sous haute pression. Le produit semi-fini ainsi obtenu est ensuite amené à la forme souhaitée par usinage mécanique, par exemple par fraisage. Enfin, afin de rendre le matériau poreux, le mélange est chauffé à 80°C. Ceci a pour effet de faire fondre le bicarbonate d'ammonium qui s'échappe et laisse place aux pores souhaités.

Une variante de ce dernier procédé consiste à fritter le mélange en le portant à une température de 1300°C, un tel frittage ayant pour effet de faire migrer les atomes et de lier les noyaux de titane entre eux, ce qui garantit ainsi la solidité de l'implant.

Si les implants obtenus par la mise en oeuvre de ces nouveaux procédés sont assurément poreux, il n'en reste pas moins qu'une fois encore il est difficile de déterminer par avance la taille et la forme des pores, et donc de réaliser un implant dont la structure est d'une porosité aussi voisine que possible de celle de l'os.

De nouveaux procédés récemment apparus permettent de ce point de vue d'obtenir des résultats beaucoup plus satisfaisants.

Pour premiers exemples, on utilise un procédé de fusion d'une poudre de titane à l'aide d'un faisceau d'électrons ou d'un faisceau laser. Ces deux procédés sont similaires : seule la source d'énergie utilisée diffère, l'énergie étant apportée par les électrons dans le premier cas et par des photons dans le second cas. Dans ces deux procédés, on dépose au fond d'un bac une couche de poudre de titane qui est fusionnée localement aux endroits souhaités. Pour cela, la géométrie de la pièce à réaliser est virtuellement découpée en tranches. La première couche de poudre correspondant à la première tranche est déposée. Le faisceau d'électrons ou le faisceau laser parcourt cette couche afin de fusionner la poudre de titane selon la géométrie de la pièce à obtenir. Une nouvelle couche de poudre est ensuite déposée sur celle existante et déjà traitée et cette nouvelle couche est également fusionnée aux endroits nécessaires. Le processus est ainsi répété jusqu'à obtention de la pièce finale par l'empilement de couches fusionnées les unes avec les autres (Acta Biomaterialia 4 (2008) 1536-1544 pour ce qui est de la fusion de poudre de titane par faisceau d'électrons et Biomaterials 27 (2006) 955-963 pour ce qui est de la fusion par faisceau laser).

Un procédé légèrement différent du précédent, dénommé LENS (Laser Engineered Net Shaping), permet en effet de réaliser également des pièces par superposition de couches de poudre de titane. Dans ce procédé LENS, la poudre est ici apportée au sein d'une tuyère qui expulse la poudre de titane à l'endroit exact où est dirigé le faisceau laser. On projette alors directement le titane en fusion selon la géométrie de la pièce à fabriquer (Acta Biomaterialia 3 (2007) 1007-1018).

L'obtention d'implants par la mise en oeuvre du procédé LENS est également décrite dans la demande de brevet internationale publiée sous la référence WO 2008/143661.

Dans tous les procédés rappelés ci-dessus, la poudre de titane qui n'est pas fusionnée est éliminée en fin de procédé afin que le produit fabriqué soit « ouvert », c'est-à-dire qu'il comporte entre les endroits fusionnés des pores et des connexions entre pores qui reproduisent aussi fidèlement que possible les caractéristiques de l'os.

Un exemple de structure poreuse permettant la réalisation d'implants chirurgicaux est décrit dans la demande de brevet US 2005/112397 publiée le 26 mai 2005 au nom de Jonathan L. Rolfe et autres. Cette structure poreuse est obtenue par la superposition de couches d'un matériau tel le titane, couches qui sont déposées successivement et qui sont alors fusionnées chacune à la couche précédente et à la couche immédiatement suivante. Chaque couche présente plusieurs ouvertures traversantes, lesquelles ouvertures se correspondent verticalement d'une couche à l'autre de telle sorte que, finalement, la structure poreuse présente une multiplicité de cheminées verticales. Toutefois, puisque ces cheminées sont donc par construction parallèles les unes aux autres, il ne peut par conséquent exister aucune connexion entre elles. La structure poreuse ainsi obtenue n'a alors qu'une porosité dans une seule dimension, la dimension verticale si l'on se réfère au mode de fabrication, et elle est ainsi bien loin de reproduire la structure osseuse, laquelle présente une porosité dans les trois dimensions afin de faciliter le plus largement possible l'intégration du tissu osseux.

Idéalement, un implant chirurgical fabriqué en titane poreux dont la structure est à motif contrôlé, répété dans l'espace, dans les trois dimensions, obtenu à partir de l'un des derniers procédés précités, par exemple le procédé LENS, doit comporter un taux de porosité d'environ 60%, une taille de pores de l'ordre de 630µm et une taille de connexions entre pores de 145µm afin que la colonisation par l'os soit optimale.

Outre ces propriétés, un implant de cette nature doit présenter des caractéristiques mécaniques de résistance et de rigidité adaptées à son usage et à son environnement. On a ainsi analysé par ordinateur plusieurs formes possibles de motifs, analyses préliminaires scientifiques à la suite desquelles il a été tiré la conclusion que, théoriquement, le motif en forme de dodécaèdre rhombique se révèlerait être le plus performant.

>

Les simulations numériques sur le motif en forme de dodécaèdre rhombique étaient en effet très concluantes. Un tel motif était théoriquement assez facilement réalisable de par ses propriétés géométriques, il permettait a priori une bonne colonisation de l'implant par l'os du fait de ses nombreuses connexions entre pores et il présentait des caractéristiques mécaniques proches de celles de l'os cortical humain.

A la suite de ces résultats théoriques encourageants, la demanderesse de la présente demande de brevet a réalisé des échantillons cubiques de 10 mm de côté en utilisant le procédé décrit ci-dessus de fusion sélective par faisceau laser et elle a analysé les échantillons en question.

Il s'avère que les résultats obtenus dans la réalité sont très éloignés des résultats théoriques. En premier lieu, la géométrie du dodécaèdre rhombique ainsi fabriqué est en fait très accidentée en ce sens que les poutres ou branches du dodécaèdre rhombique fusionnent mal. De nombreux défauts dans ces poutres, voire des discontinuités, s'observent facilement au microscope électronique. En second lieu, les propriétés mécaniques de la structure sont très faibles relativement aux propriétés mécaniques prévues théoriquement. Ainsi, dès le début des essais mécaniques de compression, on note l'apparition d'un tassement et d'une déformation permanente de la structure. Après décharge de la contrainte, les échantillons analysés ne retrouvent pas leur forme d'origine quelles que soient les déformations qu'ils ont supportées. Autrement dit, la ruine de la structure s'amorce dès l'application d'une force, aussi petite soit cette dernière. Le matériau subit une déformation irréversible, il se tasse et se raidit. Par ailleurs, les résultats mécaniques relevés, notamment le module d'Young, sont très mauvais, inférieurs de l'ordre de 40% à 60% de ceux calculés théoriquement.

Le modèle numérique idéal en forme de dodécaèdre rhombique est donc loin d'être parfait. C'est la structure elle-même qui explique les faibles propriétés mécaniques obtenues. En outre, toujours du fait de la géométrie du motif, notamment celle des poutres, il est impossible dans la réalité de fabriquer correctement un tel motif avec une finesse suffisante.

Tout ceci a conduit la demanderesse à élaborer un autre motif dont la géométrie permettrait à la fois de mieux convenir au procédé de fabrication et d'engendrer des propriétés mécaniques mieux adaptées que celles du dodécaèdre rhombique.

>

La présente invention a donc pour objet une structure poreuse à motif contrôlé, répété dans l'espace, dans les trois dimensions, ladite structure poreuse permettant la réalisation d'implants chirurgicaux destinés à combler les défects osseux caractérisée en ce que ledit motif est constitué de trois ailes disposées en étoile, chaque angle formé entre deux ailes étant sensiblement égal à 120°, chaque aile étant de forme générale rectangulaire et étant évidée en son centre.

Avantageusement, une telle structure poreuse à motif contrôlé, répété dans l'espace, est remarquable en ce que, à son extrémité libre ou pointe, chacune des trois ailes du motif est biseautée.

Dans cette construction, la largeur de la base du biseau formé à l'extrémité libre de chacune des trois ailes du motif est de préférence légèrement supérieure à l'épaisseur de ladite aile.

La structure poreuse à motif contrôlé, répété dans l'espace, définie ainsi qu'il vient d'être dit est avantageusement obtenue par l'assemblage de couches constituées chacune d'une pluralité de motifs qui, vus en plan, sont en contact les uns avec les autres par les flancs des biseaux formés à leurs pointes, et plus précisément elle est obtenue par la superposition successive de telles couches.

En outre, il est possible de faire varier les propriétés mécaniques et la porosité d'une telle structure en modifiant l'une des dimensions de ses ailes et/ou l'une des dimensions de ses évidements prévus au centre desdites ailes, et ce sans changer la forme globale du motif.

Pour premiers exemples, la dimension variable est la hauteur et/ou la largeur et/ou l'épaisseur de chaque aile.

Pour deuxièmes exemples, la dimension variable est la hauteur et/ou la largeur de chaque évidement.

Pour troisièmes exemples, la dimension variable est la hauteur et/ou la largeur et/ou l'épaisseur de chaque aile et/ou la hauteur et/ou la largeur de chaque évidement.

De manière très préférentielle, la structure poreuse à motif contrôlé, répété dans l'espace, selon l'invention est remarquable en ce qu'elle est réalisée en titane.

Les spécifications détaillées de l'invention sont données dans la description qui suit en liaison avec les dessins ci-annexés. Il est à noter que ces dessins n'ont d'autre but que celui d'illustrer le texte de la description et qu'ils ne constituent en aucune sorte une limitation de la portée de ladite invention.

La figure 1 représente, respectivement en vue de face, en vue de côté et en perspective, le dodécaèdre rhombique dont la théorie prétend qu'il serait le motif idéal.

La figure 2 représente, respectivement en vue de dessus, en vue de côté et en vue de face, le motif en forme d'étoile proposé selon la présente invention.

La figure 3 représente en vue de dessus un assemblage de six motifs de base, lequel assemblage a l'aspect d'un flocon.

La figure 4 représente, respectivement en vue de dessus, en vue de côté et en vue de face, un assemblage de plusieurs motifs de base.

La figure 5 est une vue en trois dimensions de l'assemblage représentée à la figure 4.

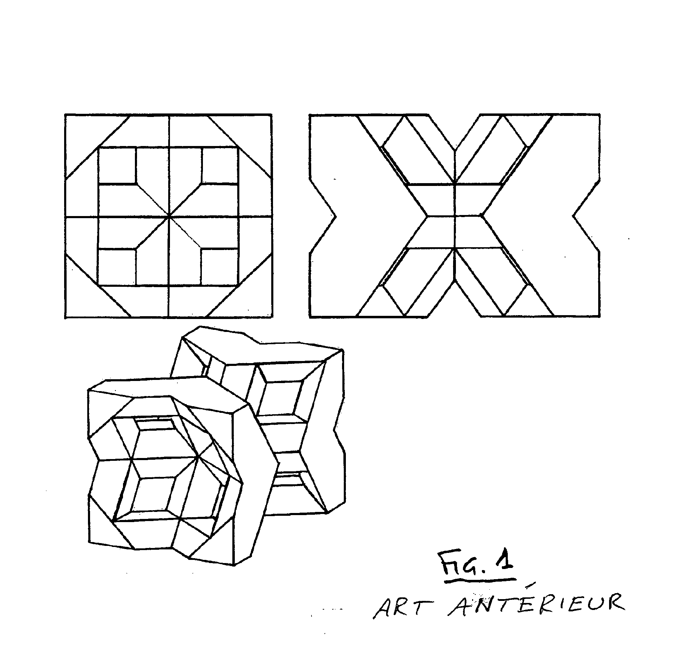

Il a été représenté à la Figure 1 le motif idéal défini par la théorie, à savoir le motif en forme de dodécaèdre rhombique. Dans ce motif idéal, les multiples espaces laissés entre les poutres ou branches du dodécaèdre rendent ce dernier indiscutablement « ouvert » et sont censées reproduire les pores et connexions entre pores de l'os.

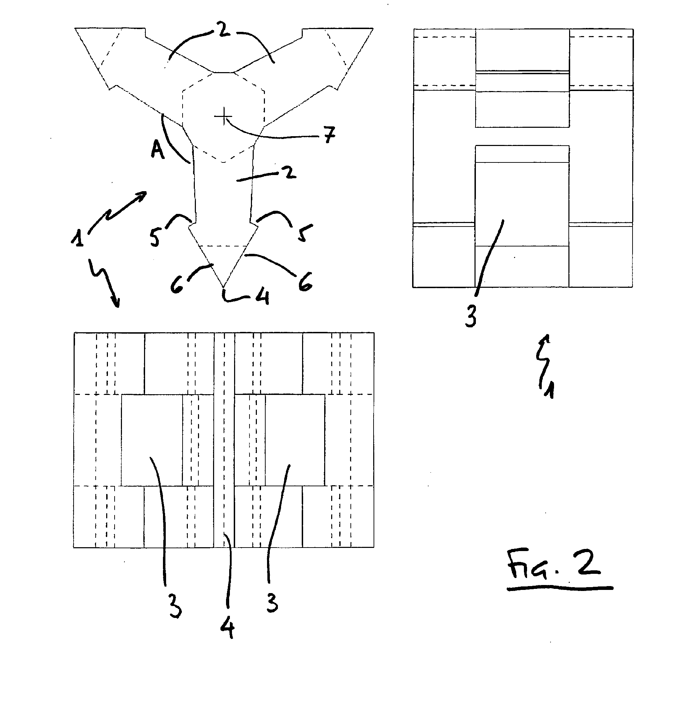

Toutefois, ainsi qu'il a été observé dans la réalité, sur des constructions de dodécaèdres rhombiques fabriqués selon les préconisations de la technique, ce motif s'avère donner de piètres résultats et être de fabrication particulièrement délicate, ce qui a conduit la demanderesse à rechercher d'autres motifs et, après études des résultats sur les autres produits ainsi réalisés, à arrêter son choix sur le meilleur produit tel qu'elle l'a défini ci-dessus et qui est représenté en détail à la Figure 2.

Ce motif 1 de base est constitué pour l'essentiel de trois ailes 2 disposées en étoile, chaque angle A formé entre deux ailes 2 étant sensiblement égal à 120°.

Chaque aile 2 est de forme essentiellement rectangulaire et elle est évidée en son centre de façon à constituer une fenêtre ou évidement 3.

A son extrémité libre, c'est-à-dire à sa pointe 4, chacune des trois ailes 2 du motif 1 est biseautée et la largeur de la base 5 du biseau est légèrement supérieure à l'épaisseur de chaque aile 2 afin de constituer deux redans.

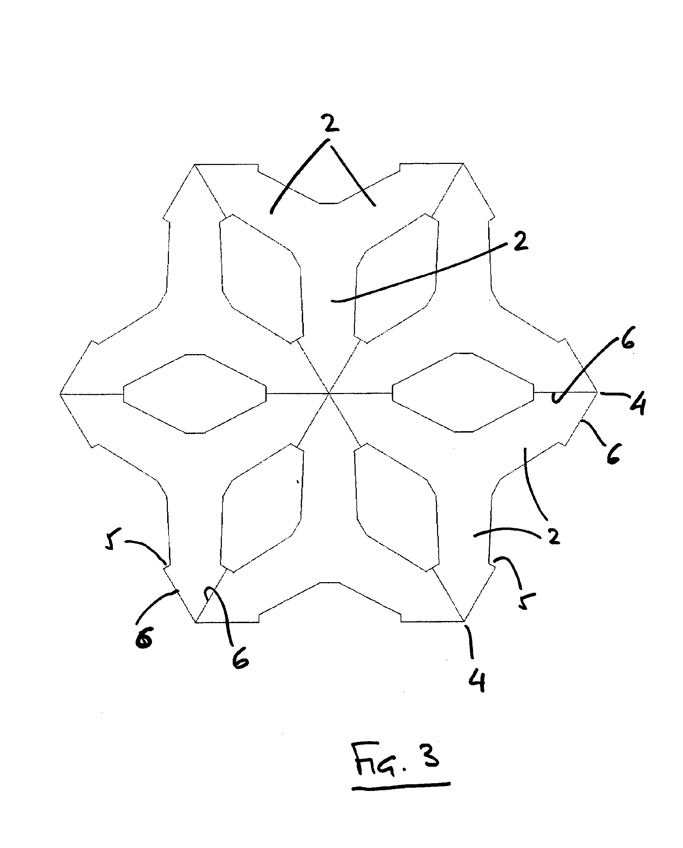

Un tel motif de base est obtenu, et répété de façon multiple dans les deux dimensions du plan, selon la technique connue dite du procédé de fusion par un faisceau d'électrons ou par un faisceau laser, en déposant une première couche de poudre de titane dans le fond d'un bac. Il est à observer que l'assemblage de six motifs de base 1, visible à la Figure 3, a ainsi l'aspect d'un flocon, les motifs étant en contact les uns avec les autres par les flancs 6 des biseaux formés à leurs différentes pointes 4. Pour que les motifs 1 soient en contact par les flancs de leurs pointes 4, il est évident que l'angle que forment entre eux les deux flancs 6 de chaque biseau est égal à 60°.

Lorsque la première couche de poudre de titane a été complètement fusionnée selon la géométrie de la pièce à obtenir, on dépose sur cette première couche une nouvelle couche de poudre que l'on fusionne à son tour aux endroits nécessaires, afin une nouvelle fois de reproduire en chaque circonstance le motif de base, et ceci tout en fusionnant les motifs de la deuxième couche à ceux de la première et en respectant à nouveau la géométrie de la pièce à obtenir.

La pièce voulue est obtenue en répétant l'opération précitée, par la superposition successive de telles couches qui sont en outre toujours fusionnées entre elles.

La forme particulière retenue à titre de motif de base a au surplus l'avantage de permettre de modifier à la fois la taille des pores et les propriétés mécaniques de la pièce finie par simple variation d'une ou de plusieurs dimensions des ailes du motif et/ou des évidements 3 prévus de fabrication dans les ailes 2 dudit motif, la forme globale de ce dernier étant en revanche sans changement.

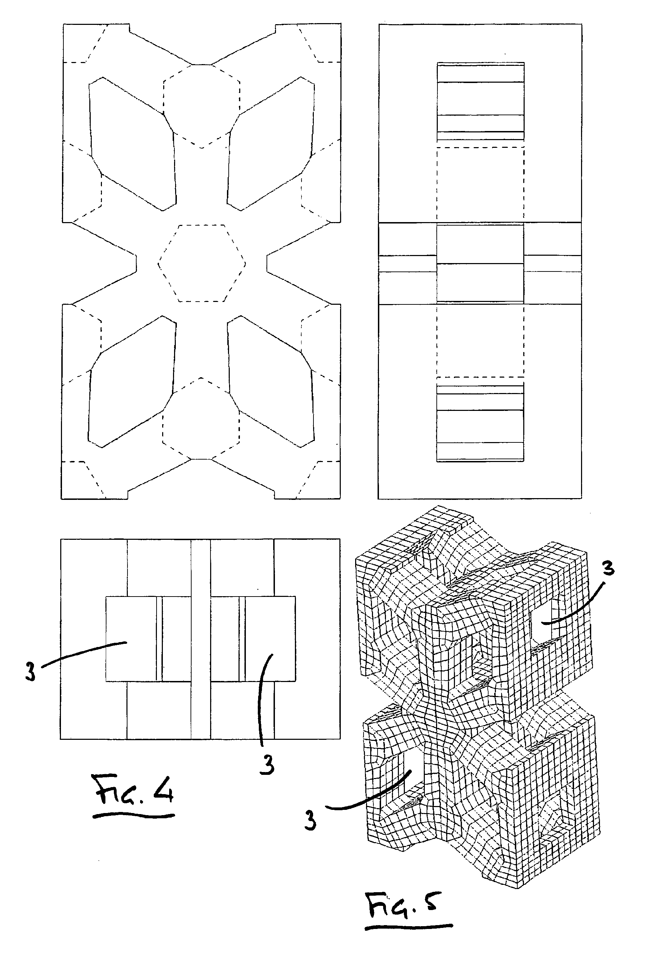

La figure 5 donne un aperçu de la pièce finale, les multiples motifs de base assemblés générant une structure qui permet de paver l'espace par des répétitions, uniquement dans les trois directions principales.

Le motif en étoile à trois ailes tel que représenté et défini ci-dessus en fait, à la connaissance de la demanderesse, le candidat le mieux adapté à la réalisation d'un substitut osseux. En effet, les propriétés mécaniques qui en découlent sont celles qui sont les plus proches de l'os, comparées aux propriétés des autres motifs que la littérature avait jusqu'ici envisagés.

A titre de simple exemple, le motif 1 en étoile à trois ailes est réalisé de façon à respecter les dimensions suivantes :
- épaisseur des ailes 2 : de l'ordre de 0,50 à 0,60 mm ;
- distance entre deux pointes 4 d'un même motif : de l'ordre de 2,80 mm ;
- distance entre une pointe 4 et l'axe principal ou axe vertical 7 du motif : de l'ordre de 1,60 mm ;
- hauteur du motif : de l'ordre de 2 à 3 mm ;
- hauteur de chaque évidement 3 : de l'ordre de 0,80 à 1,80 mm ;
- largeur de chaque évidement 3 : de l'ordre de 0,75 à 0,90 mm ;
- largeur de la base du biseau : de l'ordre de 0,65 à 0,75 mm.

## Revendications

1. Structure poreuse à motif (1) contrôlé, répété dans l'espace, dans les trois dimensions, ladite structure poreuse permettant la réalisation d'implants chirurgicaux destinés à combler les défects osseux **caractérisée en ce que** ledit motif (1) est constitué de trois ailes (2) disposées en étoile, chaque angle (A) formé entre deux ailes étant sensiblement égal à 120°, chaque aile étant de forme générale rectangulaire et étant évidée (3) en son centre.

2. Structure poreuse à motif (1) contrôlé, répété dans l'espace, selon la revendication 1, **caractérisée en ce que**, à son extrémité libre ou pointe (4), chacune des trois ailes (2) du motif est biseautée.

3. Structure poreuse à motif (1) contrôlé, répété dans l'espace, selon la revendication 2, **caractérisée en ce que** la largeur de la base (5) du biseau formé à l'extrémité libre (4) de chacune des trois ailes (2) du motif est légèrement supérieure à l'épaisseur de ladite aile.

4. Structure poreuse à motif (1) contrôlé, répété dans l'espace, selon l'une quelconque des revendications 1 à 3, **caractérisée en ce qu'**elle est obtenue par l'assemblage de couches constituées chacune d'une pluralité de motifs qui, vus en plan, sont en contact les uns avec les autres par les flancs (6) des biseaux formés à leurs pointes (4).

5. Structure poreuse à motif (1) contrôlé, répété dans l'espace, selon la revendication 4, **caractérisée en ce qu'**elle est obtenue par la superposition successive de telles couches.

6. Structure poreuse à motif (1) contrôlé, répété dans l'espace, selon l'une quelconque des revendications 1 à 5, **caractérisée en ce que** l'on fait varier ses propriétés mécaniques et sa porosité en modifiant l'une des dimensions des ailes (2) et/ou l'une des dimensions des évidements (3) prévus au centre desdites ailes, et ce sans changer la forme globale du motif.

7. Structure poreuse à motif (1) contrôlé, répété dans l'espace, selon la revendication 6, **caractérisée en ce que** la dimension variable est la hauteur et/ou la largeur et/ou l'épaisseur de chaque aile (2).

8. Structure poreuse à motif (1) contrôlé, répété dans l'espace, selon la revendication 6, **caractérisée en ce que** la dimension variables est la hauteur et/ou la largeur de chaque évidement (3).

9. Structure poreuse à motif (1) contrôlé, répété dans l'espace, selon l'une quelconque des revendications 1 à 8, **caractérisée en ce que** la dimension variable est la hauteur et/ou la largeur et/ou l'épaisseur de chaque aile (2) et/ou la hauteur et/ou la largeur de chaque évidement (3).

10. Structure poreuse à motif (1) contrôlé, répété dans l'espace, selon l'une quelconque des revendications 1 à 9, **caractérisée en ce qu'**elle est réalisée en titane.

## Patentansprüche

1. Poröse Struktur mit kontrolliertem Motiv (1), das sich im Raum in den drei Dimensionen wiederholt, wobei die poröse Struktur die Herstellung von chirurgischen Implantaten ermöglicht, die dazu bestimmt sind, Knochendefekte auszugleichen, **dadurch gekennzeichnet, dass** das Motiv (1) von drei sternförmig angeordneten Flügeln (2) gebildet ist, wobei jeder Winkel (A), der zwischen zwei Flügeln gebildet ist, im Wesentlichen gleich 120° ist, wobei jeder Flügel von allgemeiner rechteckiger Form und in seiner Mitte ausgehöhlt (3) ist.

2. Poröse Struktur mit kontrolliertem Motiv (1), das sich im Raum wiederholt, nach Anspruch 1, **dadurch gekennzeichnet, dass** jeder der drei Flügel (2) des Motivs an seinem freien Ende oder seiner Spitze (4) abgeschrägt ist.

3. Poröse Struktur mit kontrolliertem Motiv (1), das sich im Raum wiederholt, nach Anspruch 2, **dadurch gekennzeichnet, dass** die Breite der Basis (5) der am freien Ende (4) jedes der drei Flügel (2) des Motivs gebildeten Schräge leicht größer als die Dicke des Flügels ist.

4. Poröse Struktur mit kontrolliertem Motiv (1), das sich im Raum wiederholt, nach einem der Ansprüche 1 bis 3, **dadurch gekennzeichnet, dass** sie durch den Zusammenbau von Schichten hergestellt wird, die jeweils von einer Vielzahl von Motiven gebildet sind, die in Planansicht miteinander mittels der Seiten (6) der an ihren Spitzen (4) gebildeten Schrägen in Kontakt sind.

5. Poröse Struktur mit kontrolliertem Motiv (1), das sich im Raum wiederholt, nach Anspruch 4, **dadurch gekennzeichnet, dass** sie durch die aufeinanderfolgende Übereinanderlagerung solcher Schichten erhalten wird.

6. Poröse Struktur mit kontrolliertem Motiv (1), das sich im Raum wiederholt, nach einem der Ansprüche 1 bis 5, **dadurch gekennzeichnet, dass** ihre mechanischen Eigenschaften und ihre Porosität variiert werden, wobei eine der Abmessungen der Flügel (2) und/oder eine der Abmessungen der Aushöhlungen (3), die in der Mitte der Flügel vorgesehen sind, verändert wird, ohne die globale Form des Motivs zu verändern.

7. Poröse Struktur mit kontrolliertem Motiv (1), das sich im Raum wiederholt, nach Anspruch 6, **dadurch gekennzeichnet, dass** die variable Abmessung die Höhe und/oder die Breite und/oder die Dicke jedes Flügels (2) ist.

8. Poröse Struktur mit kontrolliertem Motiv (1), das sich im Raum wiederholt, nach Anspruch 6, **dadurch gekennzeichnet, dass** die variable Abmessung die Höhe und/oder die Breite jeder Aushöhlung (3) ist.

9. Poröse Struktur mit kontrolliertem Motiv (1), das sich im Raum wiederholt, nach einem der Ansprüche 1 bis 8, **dadurch gekennzeichnet, dass** die variable Abmessung die Höhe und/oder die Breite und/oder die Dicke jedes Flügels (2) und/oder die Höhe und/oder die Breite jeder Aushöhlung (3) ist.

10. Poröse Struktur mit kontrolliertem Motiv (1), das sich im Raum wiederholt, nach einem der Ansprüche 1 bis 9, **dadurch gekennzeichnet, dass** sie aus Titan hergestellt ist.

## Claims

1. Porous structure having a controlled pattern (1) which is repeated in space, in three dimensions, the porous structure allowing surgical implants which are intended to overcome bone defects to be produced, **characterised in that** the pattern (1) is constituted by three wings (2) which are arranged in the form of a star, each angle (A) formed between two wings being substantially equal to 120°, each wing being generally rectangular and being recessed (3) at the centre thereof.

2. Porous structure having a controlled pattern (1) which is repeated in space according to claim 1, **characterised in that**, at the free end or tip (4) thereof, each of the three wings (2) of the pattern is chamfered.

3. Porous structure having a controlled pattern (1) which is repeated in space according to claim 2, **characterised in that** the width of the base (5) of the chamfer formed at the free end (4) of each of the three wings (2) of the pattern is slightly greater than the thickness of the wing.

4. Porous structure having a controlled pattern (1) which is repeated in space according to any one of claims 1 to 3, **characterised in that** it is obtained by assembling layers which are each constituted by a plurality of patterns which, when viewed from above, are in contact with each other via the flanks (6) of the chamfers formed at the tips (4) thereof.

5. Porous structure having a controlled pattern (1) which is repeated in space according to claim 4, **characterised in that** it is obtained by the successive superimposition of such layers.

6. Porous structure having a controlled pattern (1) which is repeated in space according to any one of claims 1 to 5, **characterised in that** the mechanical properties and the porosity thereof are varied by modifying one of the dimensions of the wings (2) and/or one of the dimensions of the recesses (3) provided at the centre of the wings, without changing the overall shape of the pattern.

7. Porous structure having a controlled pattern (1) which is repeated in space according to claim 6, **characterised in that** the variable dimension is the height and/or the width and/or the thickness of each wing (2).

8. Porous structure having a controlled pattern (1) which is repeated in space according to claim 6, **characterised in that** the variable dimension is the height and/or the width of each recess (3).

9. Porous structure having a controlled pattern (1) which is repeated in space, according to any one of claims 1 to 8, **characterised in that** the variable dimension is the height and/or the width and/or the thickness of each wing (2) and/or the height and/or the width of each recess (3).

10. Porous structure having a controlled pattern (1) which is repeated in space according to any one of claims 1 to 9, **characterised in that** it is produced from titanium.
